# EUROPÄISCHE PATENTANMELDUNG

(11) **EP 3 431 461 A1**
(43) Veröffentlichungstag der Anmeldung: **23.01.2019**
(21) Anmeldenummer: 17182488.1
(22) Anmeldetag: 21.07.2017
(51) Int. Cl.: C07C 2/84, C07C 11/04

(54) **VERFAHREN UND ANLAGE ZUR HERSTELLUNG VON OLEFINEN**

(71) Anmelder: Linde Aktiengesellschaft, 80331 München (DE)
(72) Erfinder: NOLD, Michael, 82515 Wolfratshausen (DE); SCHMIDT, Gunther, 82041 Deisenhofen (DE)
(74) Vertreter: Frommberger, Moritz

(57) **Zusammenfassung**

Die vorliegende Erfindung betrifft ein Verfahren zur Herstellung von Olefinen, bei dem einer ersten Reaktionszone (11) eines Reaktors (10), die mit einem zur oxidativen Kopplung von Methan befähigten Katalysator ausgestattet ist, ein zumindest Methan und Sauerstoff enthaltendes Einsatzgemisch zugeführt wird, und bei dem der ersten Reaktionszone (11) ein Produktgemisch entnommen wird und in eine zweite, katalysatorfreie Reaktionszone (12) des Reaktors (10) überführt wird, wobei dem Produktgemisch zwischen der ersten Reaktionszone (11) und der zweiten Reaktionszone (12) ein kohlenwasserstoffhaltiges Gas zugespeist wird. Es ist vorgesehen, dass die Zuspeisung des kohlenwasserstoffhaltigen Gases unter Verwendung von mehreren Einspeisedüsen (14) vorgenommen wird, die um einen Umfang eines Übergangsbereichs (13) zwischen der ersten Reaktionszone (11) und der zweiten Reaktionszone (12) verteilt angeordnet sind und jeweils eine Ausströmrichtung des kohlenwasserstoffhaltigen Gases in den Übergangsbereich (13) definieren. Eine entsprechende Anlage (100) ist ebenfalls Gegenstand der vorliegenden Erfindung.

## Beschreibung

Die vorliegende Erfindung betrifft ein Verfahren zur Herstellung von Olefinen und eine entsprechende Anlage gemäß den Oberbegriffen der jeweiligen unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet. Der eingesetzte Sauerstoff wird bei den genannten Reaktionen typischerweise vollständig umgesetzt.

Die Reaktionsbedingungen bei der oxidativen Kopplung von Methan umfassen klassischerweise eine Temperatur von 500 bis 900 °C, einen Druck von 5 bis 10 bar und hohe Raumgeschwindigkeiten. Jüngere Entwicklungen gehen insbesondere auch in Richtung der Verwendung tieferer Temperaturen. Die Reaktion kann homogen- und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden, der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Typischerweise werden nicht mehr als 10 bis 15% des eingesetzten Methans umgesetzt. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

Wenngleich den geringen Ausbeuten und der Bildung von Kohlenmonoxid und Kohlendioxid teilweise durch die Wahl optimierter Katalysatoren und angepasster Reaktionsbedingungen entgegengewirkt werden kann, enthält ein bei der oxidativen Kopplung von Methan gebildetes Gasgemisch neben den Zielverbindungen wie Ethylen und ggf. Propylen überwiegend nicht umgesetztes Methan sowie Kohlendioxid, Kohlenmonoxid und Wasser. Durch ggf. erfolgende nichtkatalytische Spaltreaktionen können auch beträchtliche Mengen an Wasserstoff enthalten sein. Ein derartiges Gasgemisch wird im hier verwendeten Sprachgebrauch auch als "Produktgemisch" der oxidativen Kopplung von Methan bezeichnet, obwohl es überwiegend nicht die gewünschten Produkte, sondern auch das nicht umgesetzte Edukt Methan und die soeben erläuterten Nebenprodukte enthält.

Bei der oxidativen Kopplung von Methan können Reaktoren eingesetzt werden, in denen einer katalytischen Zone eine nichtkatalytische Zone nachgeschaltet ist. Das aus der katalytischen Zone ausströmende Gasgemisch wird in die nichtkatalytische Zone überführt, wo es zunächst noch auf den vergleichsweise hohen Temperaturen vorliegt, die in der katalytischen Zone eingesetzt werden. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann. Ein derartiges gezieltes Dampfspalten in einer der katalytischen Zone nachgeschalteten nichtkatalytischen Zone wird auch als "Post Bed Cracking" bezeichnet. Nachfolgend wird hierfür auch der Begriff "postkatalytisches Dampfspalten" verwendet.

Bezüglich den beim Dampfspalten herrschenden Reaktionsbedingungen sei ebenfalls auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Die vorliegende Erfindung stellt sich die Aufgabe, die oxidative Kopplung von Methan mit nachgeschaltetem postkatalytischem Dampfspalten gegenüber dem Stand der Technik zu verbessern.

### Offenbarung der Erfindung

Vor diesem Hintergrund schlägt die vorliegende Erfindung ein Verfahren zur Herstellung von Olefinen und eine entsprechende Anlage mit den Merkmalen der jeweiligen unabhängigen Patentansprüche vor. Ausgestaltungen sind jeweils Gegenstand der abhängigen Patentansprüche sowie der nachfolgenden Beschreibung.

Die vorliegende Erfindung geht von einem Verfahren zur Herstellung von Olefinen aus, bei dem einer ersten Reaktionszone eines Reaktors, die mit einem zur oxidativen Kopplung von Methan befähigten Katalysator ausgestattet ist, ein zumindest Methan und Sauerstoff enthaltendes Einsatzgemisch zugeführt wird, und bei dem der ersten Reaktionszone ein Produktgemisch entnommen wird und in eine zweite, katalysatorfreie Reaktionszone des Reaktors überführt wird. In der ersten Reaktionszone erfolgt dabei aufgrund ihrer Ausstattung mit dem genannten Katalysator eine oxidative Kopplung eines Teils des in dem Einsatzgemischs enthaltenen Methans in der zuvor erläuterten Weise.

Aufgrund der bei der oxidativen Kopplung von Methan verwendeten Reaktionsbedingungen tritt das Produktgemisch aus der ersten Reaktionszone mit einer Temperatur von typischerweise 800 bis 1.000 °C aus. Soll nun einen postkatalytisches Dampfspalten vorgenommen werden, wie dies insbesondere in der zweiten, katalysatorfreien Reaktionszone des im Rahmen der vorliegenden Erfindung eingesetzten Reaktors erfolgt, ist es insbesondere von Vorteil, wenn die Einmischung der stromab der ersten Reaktionszone eingespeisten zusätzlichen Kohlenwasserstoffe bei denen es sich insbesondere um Paraffine, insbesondere Ethan, Propan, Butan, Pentan, Hexan, Oktan und höhere Paraffine und/oder um entsprechende Mischungen in beliebigen Anteilen handeln kann, möglichst rasch und homogen in das Produktgemisch des postkatalytischen Dampfspalten erfolgt. Auf diese Weise kann die Ausbeute an Olefinen nochmals gesteigert werden, weil auf diese Weise insbesondere unerwünschte Sekundärreaktionen und damit sogenanntes Fouling bzw. eine Verkokung des Reaktors verhindert wird. Jedoch sind in dem erwähnten Einsatzszenario aufwendig gestaltete Verteilersysteme von Nachteil, da deren mechanische Festigkeit insbesondere über längere Zeiträume aufgrund der hohen Temperaturen des aus der ersten Reaktionszone austretenden Produktgemischs nur schwer bzw. mit hohem Aufwand zu gewährleisten ist.

Im Rahmen der vorliegenden Erfindung wurde nun erkannt, dass überraschenderweise eine Einspeisung der genannten Kohlenwasserstoffe bzw. entsprechender Gemische mittels Einspeisedüsen, die um einen Umfang eines Übergangsbereichs zwischen der ersten Reaktionszone und der zweiten Reaktionszone, insbesondere in der nachfolgend im Detail erläuterten Weise, angeordnet sind, besonders vorteilhaft ist und eine entsprechend homogene Einmischung ermöglicht.

Die vorliegende Erfindung schlägt also vor, dem Produktgemisch zwischen der ersten Reaktionszone und der zweiten Reaktionszone ein kohlenwasserstoffhaltiges Gas zuzuspeisen und dieses insbesondere in der zweiten Reaktionszone durch postkatalytisches Dampfspalten umzusetzen. Erfindungswesentlich ist dabei insbesondere, dass die Zuspeisung des kohlenwasserstoffhaltigen Gases unter Verwendung von mehreren Einspeisedüsen vorgenommen wird, welche um einen Umfang eines Übergangsbereichs zwischen der ersten Reaktionszone und der zweiten Reaktionszone verteilt angeordnet sind und welche jeweils eine Ausströmrichtung des kohlenwasserstoffhaltigen Gases in den Übergangsbereich definieren.

Unter einer "Ausströmrichtung" wird dabei im hier verwendeten Sprachgebrauch eine Richtung verstanden, in der sich zumindest der überwiegende Anteil der Gasmoleküle des Gasgemischs bzw. sich die Gasmoleküle im Mittel bewegen. Es versteht sich, dass mittels einer entsprechenden Einspeisedüse keine Bewegung sämtlicher Gasmoleküle in einer Richtung bewirkt werden kann, da sich insbesondere ein mittels einer entsprechenden Einspeisedüse bereitgestellter Gasstrom durch Kollision mit in dem Medium, in das die Einspeisung erfolgt, vorliegenden Gasmolekülen aufweitet bzw. durch einen Impuls entsprechender Gasmoleküle abgelenkt wird. Eine entsprechende Einspeisedüse kann mehrere Düsenöffnungen, die vorzugsweise zumindest überwiegend in eine Richtung weisen, oder andere Düsenformen umfassen. Insbesondere ist eine entsprechende Einspeisedüse mit der oder den Mittelachsen der Düsenöffnung(en) in der Ausströmrichtung ausgerichtet.

Es werde beispielsweise angenommen, dass das Produktgemisch aus der ersten Reaktionszone über eine kuppelförmige Oberfläche in den Übergangsbereich eintritt. Die kuppelförmige Oberfläche ist dabei zwischen dem Übergangsbereich und der ersten Reaktionszone angeordnet. Im Rahmen der vorliegenden Erfindung wurde nun erkannt, dass es besonders vorteilhaft ist, eine Reihe von Größen, die die Einströmung des kohlenwasserstoffhaltigen Gases in den Übergangsbereich definieren, aufeinander abzustimmen, wodurch eine besonders vorteilhafte, d.h. homogene und rasche, Einmischung erzielt werden kann. Selbst bei einer nicht wie nachfolgend erläutert vorgenommenen Abstimmung der genannten Größen kann jedoch im Rahmen der vorliegenden Erfindung gegenüber dem Stand der Technik, insbesondere gegenüber ebenfalls verwendbaren, aufwendigen Einspeiseeinrichtungen, ein beträchtlicher Vorteil erzielt werden. Insbesondere kann eine erfindungsgemäß ausgebildete Einspeisevorrichtung (die nachfolgenden Erläuterungen betreffen Verfahren und Vorrichtungen in gleicher Weise) in dem zuvor erläuterten Einsatzszenario besonders dauerfest und kostengünstig erstellt werden.

Als besonders vorteilhaft hat sich erwiesen, wenn ein Durchmesser, eine Anzahl, eine Ausrichtung, eine Position und eine Eindringtiefe der Einspeisedüsen in Abhängigkeit von einer Einströmung des Produktgemischs in den Übergangsbereich und einer Dimensionierung des Übergangsbereichs vorgegeben werden. Aus den Dimensionen des Übergangsbereichs ergeben sich geeignete Ausströmungsgeschwindigkeiten und diese wiederum bestimmen Anzahl und Durchmesser der Einspeisedüsen. Mit der Ausströmungsgeschwindigkeit wird erreicht, dass der Gasstrom an die kuppelförmige Oberfläche gelangt und sich dabei soweit weitet, dass er sich mit dem Produktgemisch großflächig vermischt. Entsprechendes gilt auch für eine scheibenförmige statt einer kuppelförmigen Oberfläche. Besonders vorteilhafte Ausgestaltungen werden dabei nachfolgend erläutert, wobei, wie erwähnt, auch andere Kombinationen entsprechender Parameter in anderer als der nachfolgenden erläuterten Weise gegenüber dem Stand der Technik Vorteile bieten können.

Der Übergangsbereich ist im Rahmen der vorliegenden Erfindung insbesondere zumindest teilweise zylindrisch ausgebildet. Insbesondere handelt es sich bei dem Übergangsbereich um einen Rohrabschnitt, der die erste Reaktionszone und die zweite Reaktionszone verbindet. Auch die erste und die zweite Reaktionszone können dabei im wesentlichen oder abschnittsweise zylindrisch ausgebildet sein, wobei der Übergangsbereich eine Verjüngung darstellen kann oder die erste Reaktionszone über den Übergangsbereich ohne Verjüngung in die zweite Reaktionszone übergeht.

Insbesondere kann der Übergangsbereich im Rahmen der vorliegenden Erfindung einen Innendurchmesser von 1,5 bis 2,0 Metern, insbesondere ca. 1,7 Metern, aufweisen. Die nachfolgenden Erläuterungen betreffen einen Übergangsbereich mit einer derartigen Abmessung, wobei der Fachmann bei anderen Abmessungen beispielsweise eine Extrapolation vornehmen und dabei die erfindungsgemäßen Vorteile auch bei derartigen anderen Abmessungen erzielen kann. Insbesondere schließt der Übergangsbereich an seinem der ersten Reaktionszone abgewandten Ende an die zweite Reaktionszone an, in der ein postkatalytisches Dampfspalten durchgeführt wird, wie zuvor erläutert.

Wie erläutert, sind die Einspeisedüsen im Rahmen der vorliegenden Erfindung insbesondere um einen Umfang des Übergangsbereichs angeordnet. Vorteilhafterweise sind dabei die Einspeisedüsen in einer Bezugsebene angeordnet, die senkrecht zu einer Mittelachse des Übergangsbereichs und/oder einer Hauptströmungsrichtung des Produktgemischs durch den Übergangsbereich steht. Die Einspeisedüsen bilden also einen Ring in einer Ebene um den insbesondere zylindrisch ausgebildeten Übergangsbereich. Dies schließt jedoch nicht aus, dass weitere Einspeisedüsen für das oder ein weiteres kohlenwasserstoffhaltiges Gas, beispielsweise mit abweichender Zusammensetzung, vorgesehen sein können. Diese können beispielsweise in Form mehrere Ringe um den Übergangsbereich oder der sich anschließenden zweiten Reaktionszone, beispielsweise in unterschiedlichen Temperaturzonen, angeordnet sein.

Als besonders vorteilhaft hat sich herausgestellt, wenn die Einspeisedüsen derart angeordnet sind, dass die Ausströmrichtungen jeweils einen Winkel von 40°bis 60°, insbesondere ca. 50°, zu der Bezugsebene aufweisen, und wenn die Ausströmrichtungen jeweils auf einen Punkt gerichtet sind, der in einer Ebene liegt, die zu der Bezugsebene in Richtung der ersten Reaktionszone versetzt angeordnet ist. Hierunter sei verstanden, dass die Einspeisedüsen das kohlenwasserstoffhaltiges Gas durch eine entsprechende Ausrichtung teilweise, jedoch nicht vollständig, entgegenblasen. Ein Anfangsimpuls der Gasmoleküle, der mittels der Einspeisedüsen auf diese aufgeprägt wird, weist damit eine Richtungskomponente auf, die in Richtung des Zentrums des Übergangsbereichs oder schräg hierzu (siehe sogleich) ausgerichtet ist, sowie eine Richtungskomponente, die dem durch den Übergangsbereich strömenden Produktgemisch entgegen gerichtet ist. Damit sich ein entsprechender Anfangsimpuls einstellt, wird ein gewisser Druck benötigt. Auf diese Weise lässt sich, insbesondere im Gegensatz zu einer Einspeisung senkrecht zur Hauptströmungsrichtung des Produktgemischs oder in einem steileren oder flacheren Winkel als angegeben, eine besonders gute und rasche Einmischung erzielen.

Gemäß einer besonders bevorzugten Ausgestaltungen der vorliegenden Erfindung ist ferner vorgesehen, dass die Einspeisedüsen derart angeordnet sind, dass die jeweils definierten Ausströmrichtungen einen Winkel von 0 bis 10, insbesondere ca. 0°, zu einer durch die Mittelachse in der Bezugsebene verlaufenden Radialrichtung aufweisen. Mit anderen Worten können die Einspeisedüsen direkt auf die Mittelachse des Übergangsbereichs gerichtet sein oder, insbesondere im Rahmen von Ausrichtungstoleranzen, leicht tangential hierzu versetzt. Auf diese Weise wird eine besonders gute Verwirbelung des kohlenwasserstoffhaltigen Gases bewirkt.

Wie erwähnt, wird im Rahmen der vorliegenden Erfindung das Produktgemisch insbesondere von einer kuppelförmigen Oberfläche ausgehend, die zwischen der ersten Reaktionszone und der zweiten Reaktionszone angeordnet ist, dem Übergangsbereich zugeführt. Eine derartige kuppelförmigen Oberfläche stellt insbesondere ein Gewölbe aus einem oder mehreren keramischen Werkstoffen mit einer Vielzahl über das Gewölbe verteilten, z.B. schlitzförmigen, Durchtritten dar. Die kuppelförmige Oberfläche kann insbesondere halbkugelförmig, in Form eines Halbellipsoids oder in anderer geometrischer Form ausgebildet sein. Die Kuppel öffnet sich dabei zu dem Übergangsbereich, ihr Apex ist zu der ersten Reaktionszone ausgerichtet. Die kuppelförmige Oberfläche kann insbesondere koaxial mit dem Übergangsbereich ausgerichtet sein.

Ist eine kuppelförmige Oberfläche bereitgestellt, hat es sich als besonders vorteilhaft herausgestellt, wenn der Abstand der Einspeisedüsen zu einem der ersten Reaktionszone am nächsten liegenden Punkt der kuppelförmigen Oberfläche, also deren Apex, 1,2 bis 1,5 Meter, insbesondere ca. 1,2 Meter, beträgt. In einem derartigen Abstand kann sich aufgrund der vorhandenen Distanz zu der kuppelförmigen Oberfläche von der Einspeisestelle, insbesondere bei der erläuterten, teilweise dem Produktgemisch entgegengerichteten Einspeisung, eine besonders gute Durchmischung ausbilden.

Grundsätzlich ist es jedoch auch möglich, dass das Produktgemisch von einer scheibenförmigen Oberfläche ausgehend, die zwischen der ersten Reaktionszone und der zweiten Reaktionszone angeordnet ist, dem Übergangsbereich zugeführt wird. Eine derartige scheibenförmige Oberfläche stellt insbesondere eine perforierte Scheibe aus keramischen Werkstoff dar.

Ein Abstand der Einspeisedüsen von der scheibenförmigen Oberfläche entspricht dabei vorteilhafterweise dem zuvor erläuterten Abstand der Einspeisedüsen zu dem der ersten Reaktionszone am nächsten liegenden Punkt der kuppelförmigen Oberfläche. Auf die entsprechend genannten Vorteile wird verwiesen.

Im Rahmen der vorliegenden Erfindung hat sich als besonders vorteilhaft herausgestellt, wenn 4 bis 20, insbesondere 10, Einspeisedüsen verwendet werden. Durch die vergleichsweise geringe Anzahl können die Herstellungskosten einer entsprechenden Anlage bzw. die Betriebskosten im Rahmen eines entsprechenden Verfahrens gegenüber dem Stand der Technik verringert werden.

Besonders vorteilhaft ist es, wenn die Eindringtiefe der Einspeisedüsen in den Übergangsbereich 0,0 bis 0,1 Meter, insbesondere beispielsweise ca. 0,0 Meter, beträgt. Auf diese Weise wird vermieden, dass größere Materialbereiche den hohen Temperaturen und den gegebenenfalls ausgesprochen korrosiven Bedingungen des Produktgemischs ausgesetzt werden.

Die vorliegende Erfindung erstreckt sich auch auf eine Anlage zur Herstellung von Olefinen, die einem Reaktor mit einer ersten Reaktionszone, die mit einem zur oxidativen Kopplung von Methan befähigten Katalysator ausgestattet ist, und eine zweite, katalysatorfreien Reaktionszone aufweist, wobei die Anlage Mittel, die dafür eingerichtet sind, der ersten Reaktionszone ein zumindest Methan und Sauerstoff enthaltendes Einsatzgemisch zuzuführen, Mittel, die dafür eingerichtet sind, der ersten Reaktionszone ein Produktgemisch zu entnehmen und in die zweite, katalysatorfreie Reaktionszone zu überführen, und Mittel, die dafür eingerichtet sind, dem Produktgemisch zwischen der ersten Reaktionszone und der zweiten Reaktionszone ein kohlenwasserstoffhaltiges Gas zuzuspeisen, aufweist.

Insbesondere erfindungswesentlich für eine solche Anlage ist, dass zur Zuspeisung des kohlenwasserstoffhaltigen Gases mehrere Einspeisedüsen bereitgestellt sind, die um einen Umfang eines Übergangsbereichs zwischen der ersten Reaktionszone und der zweiten Reaktionszone verteilt angeordnet sind und jeweils eine Ausströmrichtung des kohlenwasserstoffhaltigen Gases in den Übergangsbereich definieren.

Die erläuterte Anlage sowie eine Anlage gemäß einer besonders bevorzugten Ausgestaltungen der vorliegenden Erfindung, die Mittel aufweist, die zur Durchführung eines Verfahrens eingerichtet sind, wie es zuvor erläutert wurde, profitiert von den zuvor erläuterten Vorteilen, auf die daher ausdrücklich verwiesen wird.

Die Erfindung wird in bevorzugten Ausgestaltungen unter Bezugnahme auf die beigefügten Zeichnungen näher erläutert.

### Kurze Beschreibung der Zeichnung

Figur 1 veranschaulicht eine Anlage gemäß einer Ausführungsform der Erfindung in Form eines stark vereinfachten Anlagendiagramms.
Figur 2 veranschaulicht einen Reaktor gemäß einer Ausführungsform der Erfindung in vereinfachter, schematischer Teildarstellung.

### Ausführliche Beschreibung der Zeichnung

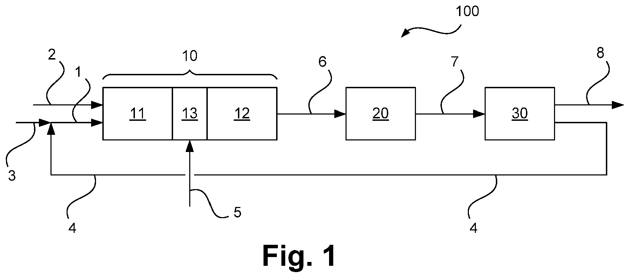

In Figur 1 ist eine Anlage gemäß einer Ausführungsform der vorliegenden Erfindung in Form eines stark vereinfachten Anlagendiagramms veranschaulicht und insgesamt mit 100 bezeichnet. Eine entsprechende Anlage 100 kann neben den in Figur 1 veranschaulichten Komponenten insbesondere eine Reihe weiterer Komponenten umfassen, die jedoch der Anschaulichkeit halber hier nicht dargestellt sind.

Als wesentliche Komponente der Anlage 100 ist ein insgesamt mit 10 bezeichneter Reaktor vorgesehen, dem im dargestellten Beispiel einen Methan enthaltener Einsatzstrom 1 und ein Sauerstoff enthaltenden Einsatzstrom 2 zugespeist werden. In dem Reaktor 10 wird auf diese Weise ein zumindest Methan und Sauerstoff enthaltendes Einsatzgemisch gebildet und zur Reaktion gebracht. Der Methan enthaltende Einsatzstrom 1 wird im dargestellten Beispiel unter Verwendung eines Frischeinsatzstroms 3 sowie eines zurückgeführten Stroms 4, dessen Herkunft unten erläutert wird, gebildet.

Der Reaktor 10 umfasst eine erste Reaktionszone 11 und eine zweite Reaktionszone 12, zwischen denen ein Übergangsbereich 13 angeordnet ist. Die erste Reaktionszone 11 ist mit einem zur oxidativen Kopplung von Methan befähigten Katalysator der eingangs erläuterten Art ausgestattet. Bei der zweiten Reaktionszone 12 handelt es sich um eine katalysatorfreie Reaktionszone, die insbesondere zum postkatalytischen Dampfspalten dient, und in die daher im dargestellten Beispiel ein kohlenwasserstoffhaltiges Gas in Form eines Stoffstroms 5 eingespeist wird. Der ersten Reaktionszone 11 wird ein in dieser gebildetes Produktgemisch entnommen und über den Übergangsbereich 13 in die zweite Reaktionszone 12 überführt.

Aufgrund der katalytischen und nichtkatalytischen Reaktionen in der ersten Reaktionszone 11 bzw. der zweiten Reaktionszone 12 des Reaktors 10 kann diesem Reaktor 10 insgesamt ein Produktgemisch in Form eines Stoffstroms 6 entnommen werden, welches anschließend insbesondere einer Konditionierung 20, umfassend beispielsweise eine Kühlung, eine Verdichtung, eine Trocknung und eine Entfernung von Sauergasen, zugeführt wird. Das entsprechend konditionierte Produktgemisch wird in Form eines Stoffstroms 7 einer Trennung 30 zugeführt, in welcher, insbesondere unter Verwendung von Tieftemperaturtrennverfahren, Membrantrennverfahren, adsorptiven Trennverfahren und dergleichen, ein oder mehrere Produkte, insbesondere Olefine wie Ethylen, abgetrennt und in Form eines oder mehrerer Produktströme 8 bereitgestellt werden können. In der Trennung 30 kann auch insbesondere in dem Reaktor 10 zuvor nicht umgesetztes Methan abgetrennt und in Form des bereits erläuterten Stoffstroms 4 dem Reaktor 10 erneut zugeführt werden.

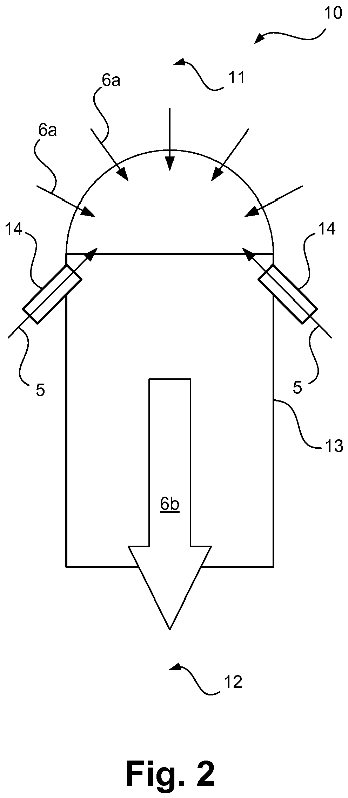

In Figur 2 ist ein Reaktor gemäß einer Ausführungsform der Erfindung in vereinfachter, schematischer Teildarstellung im Längsschnitt dargestellt und, wie bereits in Figur 1, mit 10 bezeichnet. Bereits in der Figur 1 veranschaulichte Elemente und Stoffströme sind in Figur 2 durchgängig mit identischen Bezugszeichen angegeben. Nicht im Detail veranschaulicht sind dabei die erste und die zweite Reaktionszone 11, 12, die grundsätzlich in bekannter Weise ausgebildet sein können. Die Figur 2 konzentriert sich auf die Darstellung des Übergangsbereichs 13, die erste und die zweite Reaktionszone 11, 12 sind nur angedeutet.

Wie in Figur 2 veranschaulicht, wird die Zuspeisung des kohlenwasserstoffhaltigen Gases in Form des bzw. der Stoffströme 5 unter Verwendung von mehreren Einspeisedüsen 14 vorgenommen, die um einen Umfang des Übergangsbereichs 13 zwischen der ersten Reaktionszone 11 und der zweiten Reaktionszone 12 verteilt angeordnet sind. Diese definieren jeweils eine Ausströmrichtung des kohlenwasserstoffhaltigen Gases in den Übergangsbereich.

Wie ebenfalls in Figur 2 veranschaulicht, strömt das Produktgemisch in Form der (hier nur teilweise bezeichneten) Stoffströme 6a bzw. eines gesamten Fluidstroms, der entsprechend gebildet wird, von einer kuppelförmigen Oberfläche 15 ausgehend, die zwischen der ersten Reaktionszone 11 und der zweiten Reaktionszone 13 angeordnet ist, in den Übergangsbereich 13 ein. Die Einspeisedüsen 14 sind dabei in der zuvor erläuterten Anzahl und Weise ausgebildet bzw. angeordnet. Ein stromab einer entsprechenden Einspeisung gebildetes Stoffgemisch, hier in Form eines Pfeils 6b veranschaulicht, wird in die zweite Reaktionszone 12 überführt und dort einer postkatalytischen Dampfspaltung unterworfen, wodurch letztlich der in Figur 1 veranschaulichte Stoffstrom 6 gebildet wird.

## Patentansprüche

1. Verfahren zur Herstellung von Olefinen, bei dem einer ersten Reaktionszone (11) eines Reaktors (10), die mit einem zur oxidativen Kopplung von Methan befähigten Katalysator ausgestattet ist, ein zumindest Methan und Sauerstoff enthaltendes Einsatzgemisch zugeführt wird, und bei dem der ersten Reaktionszone (11) ein Produktgemisch entnommen wird und in eine zweite, katalysatorfreie Reaktionszone (12) des Reaktors (10) überführt wird, wobei dem Produktgemisch zwischen der ersten Reaktionszone (11) und der zweiten Reaktionszone (12) ein kohlenwasserstoffhaltiges Gas zugespeist wird, **dadurch gekennzeichnet, dass** die Zuspeisung des kohlenwasserstoffhaltigen Gases unter Verwendung von mehreren Einspeisedüsen (14) vorgenommen wird, die um einen Umfang eines Übergangsbereichs (13) zwischen der ersten Reaktionszone (11) und der zweiten Reaktionszone (12) verteilt angeordnet sind und jeweils eine Ausströmrichtung des kohlenwasserstoffhaltigen Gases in den Übergangsbereich (13) definieren.

2. Verfahren nach Anspruch 1, bei ein Durchmesser, eine Anzahl, eine Ausrichtung, eine Position und eine Eindringtiefe der Einspeisedüsen (14) in Abhängigkeit von einer Einströmung des Produktgemischs in den Übergangsbereich (13) und einer Dimensionierung des Übergangsbereichs (13) vorgegeben werden.

3. Verfahren nach Anspruch 1 oder 2, bei dem der Übergangsbereich (13) zumindest teilweise zylindrisch ausgebildet ist.

4. Verfahren nach Anspruch 3, bei dem der Übergangsbereich einen Innendurchmesser von 1,5 bis 2,0 Metern aufweist.

5. Verfahren nach Anspruch 3 oder 4, bei dem die Einspeisedüsen (14) in einer Bezugsebene angeordnet sind, die senkrecht zu einer Mittelachse des Übergangsbereichs und/oder einer Hauptströmungsrichtung des Produktgemischs durch den Übergangsbereich (13) steht.

6. Verfahren nach einem der Ansprüche 3 bis 5, bei dem Einspeisedüsen (14) derart angeordnet sind, dass die Ausströmrichtungen jeweils einen Winkel von 40°bis 60°zu der Bezugsebene aufweisen, und wobei die Aus strömrichtungen jeweils auf einen Punkt gerichtet sind, der in einer Ebene liegt, die zu der Bezugsebene in Richtung der ersten Reaktionszone (11) versetzt angeordnet ist.

7. Verfahren nach einem der Ansprüche 5 oder 6, bei dem die Einspeisedüsen (14) derart angeordnet sind, dass die jeweils definierten Ausströmrichtungen einen Winkel von 0 bis 10 zu einer durch die Mittelachse in der Bezugsebene verlaufenden Radialrichtung aufweisen.

8. Verfahren nach einem der vorstehenden Ansprüche, bei dem das Produktgemisch von einer kuppelförmigen Oberfläche (15) ausgehend, die zwischen der ersten Reaktionszone (11) und der zweiten Reaktionszone (12) angeordnet ist, dem Übergangsbereich (13) zugeführt wird.

9. Verfahren nach Anspruch 8, bei dem der Abstand der Einspeisedüsen (14) von zu einem der ersten Reaktionszone (11) am nächsten liegenden Punkt der kuppelförmigen Oberfläche (15) 1,2 bis 1,5 Meter beträgt.

10. Verfahren nach einem der vorstehenden Ansprüche, bei dem das Produktgemisch von einer scheibenförmigen Oberfläche (15) ausgehend, die zwischen der ersten Reaktionszone (11) und der zweiten Reaktionszone (12) angeordnet ist, dem Übergangsbereich (13) zugeführt wird.

11. Verfahren nach Anspruch 10, bei dem der Abstand der Einspeisedüsen (14) von der scheibenförmigen Oberfläche (15) 1,2 bis 1,5 Meter beträgt.

12. Verfahren nach einem der vorstehenden Ansprüche, bei dem 4 bis 20 Einspeisedüsen (14) verwendet werden.

13. Verfahren nach einem der vorstehenden Ansprüche, bei dem die Eindringtiefe der Einspeisedüsen (14) in den Übergangsbereich (13) 0,0 bis 0,1 Meter beträgt.

14. Anlage (100) zur Herstellung von Olefinen, die einem Reaktor (10) mit einer ersten Reaktionszone (11), die mit einem zur oxidativen Kopplung von Methan befähigten Katalysator ausgestattet ist, und eine zweite, katalysatorfreien Reaktionszone (12) aufweist, wobei die Anlage Mittel, die dafür eingerichtet sind, der ersten Reaktionszone (11) ein zumindest Methan und Sauerstoff enthaltendes Einsatzgemisch zuzuführen, Mittel, die dafür eingerichtet sind, der ersten Reaktionszone (11) ein Produktgemisch zu entnehmen und in die zweite, katalysatorfreie Reaktionszone (12) zu überführen, und Mittel, die dafür eingerichtet sind, dem Produktgemisch zwischen der ersten Reaktionszone (11) und der zweiten Reaktionszone (12) ein kohlenwasserstoffhaltiges Gas zuzuspeisen, aufweist, **dadurch gekennzeichnet, dass** zur Zuspeisung des kohlenwasserstoffhaltigen Gases mehrere Einspeisedüsen (14) bereitgestellt sind, die um einen Umfang eines Übergangsbereichs (13) zwischen der ersten Reaktionszone (11) und der zweiten Reaktionszone (12) verteilt angeordnet sind und jeweils eine Ausströmrichtung des kohlenwasserstoffhaltigen Gases in den Übergangsbereich (13) definieren.

15. Anlage (100), die Mittel aufweist, die zur Durchführung eines Verfahrens nach einem der vorstehenden Ansprüche eingerichtet sind.
